(19) Europäisches Patentamt / European Patent Office / Office européen des brevets

(11) **EP 2 184 269 B1**

(12) **EUROPEAN PATENT SPECIFICATION**

(45) Date of publication and mention
of the grant of the patent:
**01.10.2014 Bulletin 2014/40**

(21) Application number: **08791988.2**

(22) Date of filing: **31.07.2008**

(51) Int Cl.:
*C07C 2/76* *(2006.01)*        *B01J 29/48* *(2006.01)*
*B01J 29/78* *(2006.01)*        *C07C 15/04* *(2006.01)*
*C10G 50/00* *(2006.01)*        *C07B 61/00* *(2006.01)*

(86) International application number:
**PCT/JP2008/063773**

(87) International publication number:
**WO 2009/020045 (12.02.2009 Gazette 2009/07)**

(54) **PROCESS FOR PRODUCTION OF AROMATIC HYDROCARBONS**

VERFAHREN ZUR HERSTELLUNG AROMATISCHER KOHLENWASSERSTOFFE

PROCÉDÉ DE PRODUCTION D'HYDROCARBURES AROMATIQUES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **03.08.2007 JP 2007203221**

(43) Date of publication of application:
**12.05.2010 Bulletin 2010/19**

(73) Proprietors:
• **Mitsui Chemicals, Inc.
Tokyo 105-7117 (JP)**
• **Agency For Science, Technology And Research
Singapore 138668 (SG)**

(72) Inventors:
• **NISHIMURA, Toru
Jurong Island 627833 (SG)**
• **AOKI, Shinobu
Sodegaura-shi
Chiba 299-0265 (JP)**
• **LIU, Yan
Jurong Island 627833 (SG)**

(74) Representative: **Raynor, Stuart Andrew et al
J A Kemp
14 South Square
Gray's Inn
London WC1R 5JJ (GB)**

(56) References cited:
WO-A1-2005/028105        WO-A1-2006/011568
JP-A- 2003 026 613        JP-A- 2004 269 398
JP-A- 2004 521 070        JP-A- 2005 255 605

**Description**

TECHNICAL FIELD

The present invention relates to a process for producing an aromatic hydrocarbon from a lower hydrocarbon gas. More particularly, the present invention relates to a process for efficiently producing an aromatic hydrocarbon useful as a chemical industrial raw material from a lower hydrocarbon gas containing methane as a major component by activating a molybdenum-containing solid catalyst.

TECHNICAL BACKGROUND

Conventionally, most of aromatic hydrocarbons represented by benzene, toluene, xylene, etc. are produced as a by-product of gasoline production in petroleum refinery industry or as a by-product of ethylene production in petrochemical industry. The yields of aromatic hydrocarbons based on crude oil, which is a starting raw material, are not high because the aromatic hydrocarbons are not targeted products in any of the industries. Furthermore, the production of aromatic hydrocarbon is limited by the demand trends of each targeted product.

As a process for producing an aromatic hydrocarbon as an targeted product, processes of employing a light cut component derived from crude oil, for example, a process of using LPG or $C_3$-$C_4$ naphtha such as Cyclar process, Z-forming process, Aroforming process and M2 forming process; a process of employing a $C_6$-$C_7$ naphtha such as AROMAX process or RZ-Platformer process and alpha process of employing cracked olefins, and other processes have been developed, and some of the processes have been commercialized. However, the production of aromatic hydrocarbon still remains low.

Meanwhile, it is said that the natural gas reserves in the whole world are 6000 TCF. However, the transportation cost of the natural gas is generally high and most of the natural gas is unevenly distributed in the limited areas. Therefore, most of the natural gas is not utilized effectively. For example, associated gas produced together with crude oil contains a large amount of methane, but recovery and transportation of methane have many problems so that methane is generally disposed by incineration. The technology for producing aromatic hydrocarbons from methane, which is a main component of natural gas, is not only a process that abundant natural gas can be converted into essentially high-value-added liquid chemical products, which is more easily transported, but also a process that a raw material source of aromatic hydrocarbon, which is one important chemical and industrial raw material, can be switched to non-crude oil resources. The commercialization of the process has been desired.

Examples of the process for producing aromatic hydrocarbons from methane include a process of producing aromatics by a multiple-step reaction and a process of producing aromatics directly by a one-step reaction. An example of the former process is a process that a water gas is produced firstly from methane, methanol is then produced from the water gas and a gasoline component containing aromatics is finally produced by MTG reaction. Alternatively, Patent document 1 and Patent document 2 disclose processes of producing a gas stream containing an aromatic hydrocarbon using a mixture essentially containing ethane and ethylene obtainable by an oxidative coupling reaction of methane as a raw material.

As a process for directly making methane into aromatics, there have been disclosed a report that methane is allowed to contact with silica to prepare 1 to 4 ring aromatic compounds (Non-Patent document 1) and reports by O. V. Bragin, *et al.* that an aromatic compound such as benzene and the like is prepared by allowing methane to contact with a catalyst comprising platinum or chromium supported on zeolite (Non-Patent document 2 and Non-Patent document 3).

Patent document 3 discloses that when methane is fed to Ca-Cr-Pt/alumina at about 700°C, ethylene, benzene and the like are obtained in small amounts. Patent documents 4 to 6 disclose that slight amounts of aromatics can be synthesized from methane using boron nitride, silicon carbide or a metal oxide, as a catalyst, at a high temperature of over 1000°C.

Patent document 7 discloses a method of producing a product containing aromatics and olefins from hydrocarbon by using a catalyst, which comprises zinc, another metal and zeolite, but no examples of employing a raw material containing methane is disclosed.

Patent document 8 discloses a method of producing a liquid rich in aromatic hydrocarbons from methane by using Ga/ZSM-5 or H-ZSM-5, as a catalyst. Patent document 9 discloses a method that benzene is produced from methane using a carbonaceous substance at least partly containing a diamond-like structure.

Patent document 10 discloses a method that a liquid rich in aromatic hydrocarbons is produced from a hydrocarbon containing methane, as a main component, using a catalyst containing a VIIB metal such as gallium, rhenium and the like and an aluminosilicate having a ratio of silica to alumina of not less than 5. Patent document 11 discloses a method that a lower hydrocarbon such as methane and the like is aromatized using a catalyst, which comprises metallosilicate and any one of zinc, gallium and cobalt. Patent document 12 discloses a method that an aromatic hydrocarbon is prepared from methane using a catalyst, which comprises gallium and a pentasil type zeolite, and an alumina modified with phosphorus in combination. Moreover, Patent document 13 disclosed a method that an aromatic hydrocarbon is produced from methane mixed with any one of C2 to C4 olefins, propane and n-hexane in an amount of 40 to 105 mol% based on methane.

Patent document 14 and Patent document 15 disclose methods that benzene and the like are prepared from a lower hydrocarbon such as methane and the like using a catalyst which comprises a porous support, rhenium and any one of iron, cobalt, platinum and the like, or a catalyst which comprises rhenium supported on metallosilicate.

As a widely known catalyst having excellent properties which has been studied most earnestly for producing an aromatic hydrocarbon using methane as a raw material, a catalyst comprising molybdenum supported on a zeolite developed by L. Wang, *et al.* in 1993 can be cited (Non-Patent document 4). This document discloses a method of using the catalyst in the reaction after treatment with air at 700°C.

Patent document 16 discloses a method of preparing a Mo/ZSM-5 catalyst by a mixing and heating process or a CVD process. However, the performance is not sufficient since the aromatic yield is low and the catalyst life is not studied sufficiently. Patent document 17 discloses a method of preparing a Mo/ZSM-5 catalyst used for accurately controlling a moisture removal process in a drying step after a supporting step. Patent document 18 discloses a method of preparing a shaped catalyst which prevents an active component from being supported onto a binder. However, according to the examples thereof, the deterioration of the catalyst activity with time progresses very rapidly and sufficient capabilities cannot be obtained.

Patent document 11, Patent document 19 and Non-Patent documents 5 to 9 disclose methods of preparing aromatics from a lower hydrocarbon such as methane and the like using a catalyst obtainable by adding, to a catalyst which comprises molybdenum and metallosilicate, any one of zinc, gallium, cobalt, chromium, lanthanum, neodymium, samarium, yttrium, iron, cobalt, nickel, platinum, zirconium, tungsten, vanadium, palladium, ruthenium, iridium, titanium, rhodium, rhenium, gold, silver and copper. However, the performance is not sufficient since the aromatic yield is low and the catalyst life is not studied sufficiently.

Patent document 20 discloses a method of aromatizing methane using a catalyst obtainable by introducing molybdenum into a MFI type zeolite having a ratio of silica to alumina of about 50, outer surface acidity of which is inactivated with an amorphous silica layer, and a process of activating the molybdenum-containing aluminosilicate catalyst in which molybdenum is introduced. Furthermore, Patent documents 21 to 23 disclose methods of preparing a molybdenum-containing catalyst by carbonization treatment.

Patent documents 24 to 28 disclose methods of improving the catalyst life by adding carbon monoxide, carbon dioxide, hydrogen or a mixture of hydrogen and water to a reaction gas in production of aromatics from methane using a catalyst which comprises molybdenum and metallosilicate. Moreover, Patent document 28 and Patent document 29 disclose methods of keeping the catalyst activity by feeding a reaction gas and a hydrogen-containing gas alternatively in production of benzene and the like from methane using a catalyst which comprises molybdenum and metallosilicate. However, in any of the methods, the improvement is effected but is not sufficient necessarily, and still the yield of the aromatics in any of the methods is low.

Patent document 30 discloses a process of preparing a non-aromatic cyclic hydrocarbon such as cyclohexane and the like by converting methane, which is mixed with at least one selected from hydrogen, water, carbon monoxide and carbon dioxide, with a Mo/ZSM-5 catalyst and subsequently allowing an effluent containing the resulting aromatics such as benzene and the like to react with hydrogen. Patent document 31 discloses a process of preparing an aromatic compound by reacting methane obtainable from reaction of hydrogen and carbon monoxide or carbon dioxide, using a Mo/ZSM-5 catalyst.

In most of these documents, the catalyst life determined by lowering of the yield of the aromatic hydrocarbon with time is insufficient from the industrial viewpoint, or necessary examinations on the catalyst life are not carried out. In some of the methods in which the catalyst life is long, the yield of the aromatic hydrocarbon is suppressed to be low level. Therefore, the development of a technique capable of keeping a high yield of the aromatic hydrocarbon for a long period of time has been desired.

Patent document 1: USP 4,822,944
Patent document 2: USP 5,336,825
Patent document 3: USP 4,239,658
Patent document 4: USP 4,507,517
Patent document 5: USP 4,567,311
Patent document 6: USP 4,734,537
Patent document 7: WO-A-98/51409
Patent document 8: EP-B-216491
Patent document 9: USP 5,557,022
Patent document 10: EP-B-228267
Patent document 11: JP-A-H10(1998)-272366

Patent document 12: USP 5,026,937
Patent document 13: USP 5,936,135
Patent document 14: USP 6,239,057
Patent document 15: JP-A-2001-334151
Patent document 16: JP-A-2002-336704
Patent document 17: JP-A-2004-97891
Patent document 18: JP-A-2005-144360
Patent document 19: JP-A-H11(1999)-47606
Patent document 20: WO-A-02/10099
Patent document 21: WO-A-2005/28105
Patent document 22: JP-A-2005-254120
Patent document 23: JP-A-2005-254121
Patent document 24: JP-A-H11(1999)-60514
Patent document 25: JP-A-2004-269398
Patent document 26: JP-A-2005-255605
Patent document 27: JP-A-2005-343879
Patent document 28: WO-A-2006/11568
Patent document 29: JP-A-2003-26613
Patent document 30: WO-A-2006/83409
Patent document 31: WO-A-2006/87971
Non-Patent document 1: Science Vol. 153 Page 1393 (1966)
Non-Patent document 2: Russian Chemical Bulletin Vol. 31 No. 4 Page 847 (1982)
Non-Patent document 3: Russian Chemical Bulletin Vol. 38 No. 3 Page 680 (1989)
Non-Patent document 4: Catalysis Letters Vol. 21 Page 35 (1993)
Non-Patent document 5: Catalysis Letters Vol. 39 Page 169 (1996)
Non-Patent document 6: Applied Catalysis A Vol. 152 Page 173 (1997)
Non-Patent document 7: Reaction Kinetics and Catalysis Letters Vol. 61 Page 391 (1997)
Non-Patent document 8: Journal of Catalysis Vol. 170 Page 11 (1997)
Non-Patent document 9: Journal of Natural Gas Chemistry Vol. 13 Page 36 (2004)

## DISCLOSURE OF THE INVENTION

### SUBJECT TO BE SOLVED BY THE INVENTION

It is an object of the present invention to provide a process for producing an aromatic hydrocarbon using a molybdenum-containing solid catalyst, more specifically to provide a process for producing an aromatic hydrocarbon efficiently from a lower hydrocarbon gas essentially containing methane by activating a molybdenum-containing solid catalyst with maintaining a high yield for a long period of time.

### MEANS FOR SOLVING THE SUBJECT

The present inventors have been earnestly studied in order to solve the above subjects and found that in producing an aromatic hydrocarbon from a lower hydrocarbon gas essentially containing methane in the presence of a molybdenum-containing solid catalyst, the molybdenum-containing catalyst is allowed to preliminarily contact with a pre-contacting gas (G1) comprising at least one selected from a lower hydrocarbon and a hydrogen gas at a temperature lower than a reaction temperature and then to contact with a lower hydrocarbon gas essentially containing methane which is a raw material gas (G2), to carry out a reaction and thereby a sufficiently high yield of the aromatic hydrocarbon can be maintained industrially for a long time period of time. Thus, the present invention has been accomplished.

That is to say, the process for producing an aromatic hydrocarbon of the present invention comprises:

a pre-contacting step of allowing a molybdenum-containing solid catalyst to contact with a pre-contacting gas (G1) comprising at least one selected from a lower hydrocarbon and a hydrogen gas; and
a reaction step of allowing the pre-contacted molybdenum-containing solid catalyst to contact with a raw material gas (G2) comprising a lower hydrocarbon essentially containing methane, to generate the aromatic hydrocarbon,
wherein the starting temperature in the pre-contacting step is lower than the reaction temperature (Tr) in the reaction step, and the temperature during pre-contacting step from the beginning to the end is not over the reaction temperature (Tr), and the molybdenum-containing solid catalyst comprises molybdenum and a zeolite having a ratio of silica to alumina of not more than 45.

In the process for producing an aromatic hydrocarbon according to the present invention, the starting temperature in the pre-contacting step is preferably not higher than 350° C and the pre-contacting temperature is preferably increased with passage of time.

In the process for producing an aromatic hydrocarbon according to the present invention, in the pre-contacting step, the contact of the molybdenum-containing solid catalyst and the pre-contacting gas (G1) is preferably carried out with maintaining the temperature at a holding temperature (Th) which is lower than the reaction temperature (Tr) for a certain time. Moreover, the holding temperature (Th) in the pre-contacting step is preferably not lower than 400 °C and not higher than the reaction temperature (Tr).

In the process for producing an aromatic hydrocarbon according to the present invention, the reaction step is preferably started after when the temperature of the pre-contacting step reaches to the reaction temperature (Tr), the pre-contacting gas (G1) is changed to the raw material gas (G2).

In the process for producing an aromatic hydrocarbon according to the present invention, the pre-contacting gas (G1) is preferably a mixed gas comprising hydrogen and a lower hydrocarbon essentially containing methane, more preferably a mixed gas comprising methane and hydrogen. Moreover, the mixed gas preferably comprises 5 or more moles of hydrogen per 1 mole of methane, more preferably the mixed gas comprises 10 or more moles of hydrogen per 1 mole of methane.

In the process for producing an aromatic hydrocarbon according to the present invention, in the pre-contacting step, the flow rate of the pre-contacting gas (G1) preferably satisfies at least one condition of a condition such that the flow rate of the pre-contacting gas (G1) is more than two times as high as the flow rate of the reaction gas (G2) and a condition such that the ratio (F/W) of the flow rate (F) of the pre-contacting gas (G1) to the catalyst weight(W) is not less than 50 cc/(g·min).

In the process for producing an aromatic hydrocarbon according to the present invention, after the atmospheric gas of a catalyst layer is purged with an inert gas, the pre-contacting step is preferably carried out and further the temperature of the purging with an inert gas is more preferably not higher than 350 ° C.

In the process for producing an aromatic hydrocarbon according to the present invention, the raw material gas (G2) preferably does not contain hydrogen substantially.

In the process for producing an aromatic hydrocarbon according to the present invention, the molybdenum-containing solid catalyst preferably comprises molybdenum and a crystalline metallosilicate, the crystalline metallosilicate is preferably an MFI type zeolite or an MWW type zeolite and moreover, the molybdenum is preferably supported in an amount of 7 to 20 % by weight on the molybdenum-containing solid catalyst.

EFFECT OF THE INVENTION

The present invention can provide a process for producing an aromatic hydrocarbon efficiently from a lower hydrocarbon gas comprising methane as a main component and other natural gas by activating a molybdenum-containing solid catalyst with simple and economical procedures and thereby maintaining a high reaction yield for a long period of time in no need of changing a device configuration, a complicated catalyst modification treatment or the use of a special treatment gas. In the present invention, hydrogen can be produced together with the aromatic hydrocarbon, and thereby it is also useful as a process for producing hydrogen from methane.

BEST EMBODIMENT FOR CARRYING OUT THE INVENTION

The present invention will be described in detail below.

The process for producing an aromatic hydrocarbon of the present invention comprises:

a pre-contacting step of allowing a molybdenum-containing solid catalyst to contact with a pre-contacting gas (G1) comprising at least one selected from a lower hydrocarbon and a hydrogen gas; and
a reaction step of allowing the pre-contacted molybdenum-containing solid catalyst to contact with a raw material gas (G2) comprising a lower hydrocarbon essentially containing methane, to generate the aromatic hydrocarbon.

Pre-contacting step

In the pre-contacting step of the present invention, the molybdenum-containing solid catalyst is allowed to contact with the pre-contacting gas (G1) comprising at least one selected from a lower hydrocarbon and a hydrogen gas.

Molybdenum-containing solid catalyst

The molybdenum-containing solid catalyst of the present invention comprises molybdenum and a zeolite having a ratio of silica to alumina of not more than 45. It is possible to use a known molybdenum-containing solid catalyst prepared by a known process. That is to say, it is possible to use a molybdenum compound itself, a molybdenum metal or a molybdenum compound supported on a support or a catalyst prepared by mixing a molybdenum metal or a molybdenum compound with an inorganic solid physically. The catalyst may be calcined in the air or in an inert atmospheric gas such as nitrogen gas after supporting or mixing, and then may be submitted to use. The catalyst may be calcined preferably in the air at a temperature of 250 to 800ºC, more preferably 350 to 600°C, furthermore preferably 450 to 550ºC and submitted to use. Particularly, it is preferred to use the molybdenum compound supported on a support as the catalyst.

The amount of the molybdenum component supported or mixed is, in terms of the molybdenum metal as a percentage of the total weight of the catalyst, 1 to 50 % by weight, preferably 5 to 20 % by weight, more preferably 7 to 20 % by weight, furthermore preferably 8 to 15 % by weight, particularly preferably 10 to 12 % by weight.

There is no particular limitation on the molybdenum source. As the molybdenum source, commercially available molybdenum metals and various molybdenum compounds can be used. Examples thereof, which are easily available relatively, are molybdenum oxide, molybdenum carbide, molybdic acid, sodium molybdate, ammonium molybdate, ammonium heptamolybdate, ammonium paramolybdate, 12-molybdophosphoric acid and 12-molybdosilicic acid.

When molybdenum or the molybdenum compound is supported on the support, various known methods can be used, for example, impregnation methods such as pore filling method, incipient wetness method, equilibrium adsorption method, evaporation to dryness method or spray drying method, a deposition method, an ion exchange method and a chemical vapor deposition method. Of these methods, the impregnation methods are preferable because their procedures are relatively simple and a special device is unnecessary.

When the catalyst is used in a shaped form, it is possible to employ any one of a process of carrying out the above supporting procedure to a powdery support to prepare a supported catalyst powder and then shaping, and a process of preparing a shaped body only composed of a support and carrying out supporting of a molybdenum component on the body.

The catalyst is preferably used after calcinating thereof in the air. After supporting or the mixing of the molybdenum, and before the calcinating thereof, it is preferred to dry it sufficiently. The drying is carried out at a temperature of 80 to 150°C, more preferably 100 to 120°C overnight or for a day and night.

In addition to molybdenum, at least one other metal component can be supported or mixed in order to improve the activity, selectivity, catalyst life or ease of regeneration. Examples of the metal component are titanium, vanadium, chromium, manganese, iron, cobalt, nickel, copper, zinc, aluminum, gallium, yttrium, zirconium, ruthenium, rhodium, palladium, silver, lanthanum, neodymium, samarium, tungsten, rhenium, iridium, platinum and gold. Of these, preferred are zirconium, tungsten or rhenium, iron, cobalt or nickel (iron group), ruthenium, rhodium, palladium or platinum (platinum group) and copper, silver or zinc. In this case, there is no limitation on the order or the method of supporting or mixing the two or more components. These components can be introduced simultaneously or successively by an arbitrary method.

As the above support or the inorganic solid, it is possible to use a commercially available one as it is, or one obtainable by synthesizing from an inorganic compound raw material using a known method. Moreover, two or more supports or inorganic solids can be used in combination. As the support or inorganic solid, inorganic solids having high heat resistance are used and particularly, an oxide support is used frequently. Specifically, it is possible to use crystalline or non-crystalline oxides such as silica, alumina, titania, zirconia, magnesia, silica-alumina, silica-titania or silica-magnesia; crystalline oxides having micropores, for example, zeolites or aluminosilicates, crystalline metallosilicates illustrated by gallosilicate, galloaluminosilicate, borosilicate or phosphoaluminosilicate, and crystalline aluminophosphates; and MCM-41, MCM-48, MCM-50, SBA-15, and FSM-16 which have mesopores, and those obtainable by regulating these pore diameters with chemical modification. These pore diameters are preferably from 0.4 to 0.9 nanometer, more preferably 0.5 to 0.6 nanometer.

Among the above, particularly, crystalline metallosilicates having a FAU, LTL, BEA, MOR, FER, MFI, MTW, MEL, CHA, MTT, DON, TON, MWW, NES, MFS, STF, STT, SFG, KFI, IWR, ITH or IWW type structure are preferred, and furthermore, zeolites are preferred. Moreover, crystalline metallosilicates illustrated by the names, for example, ALPO-5, EMM-1, EMM-2, ERB-1, ETS-2, ETS-10, ITQ-1, ITQ-2, ITQ-13, ITQ-22, ITQ-23, ITQ-24, ITQ-25, ITQ-26, L zeolite, MCM-22, MCM-36, MCM-49, MCM-56, MCM-68, NU-1, NU-86, NU-87, PSH-3, SAPO-5, SAPO-11, SAPO-17, SAPO-34, SAPO-35, SAPO-41, SSZ-25, SSZ-58, USY zeolite, X zeolite, Y zeolite, VPI-5, ZK-5, ZRP-1, ZSM-5, ZSM-8, ZSM-11, ZSM-12, ZSM-22, ZSM-23, ZSM-35, ZSM-48, ZSM-57, silicalite, ferrierite, beta-zeolite, mordenite and Molecular Sieve 5A are preferred, and among them, zeolites are more preferred. Especially, MFI type zeolites typified by ZSM-5 type zeolite, and MWW type zeolites typified by MCM-22 type zeolite are preferred.

In the case of using these zeolites, the ratio of silica to alumina is preferably smaller as long as any loss of the stability of the zeolite structure is avoided, for example, preferably not more than 45, still preferably not more than 35, still further preferably not more than 30. In this case, zeolite synthesized so as to have such a ratio of silica to alumina can be used, or the ratio of silica to alumina of zeolite can be regulated to use before or after the molybdenum introduction by a known method such as dealumination and the like.

The above supports or inorganic solids preferably have solid acid properties, and known solid acids can be used as the support. In the case of using zeolite or methallosilicate, the solid can possess acid properties by using proton type or ammonium type zeolite as the raw material for the catalyst preparation or by converting other type of zeolite into proton type by a known method after the catalyst preparation.

The catalyst has no limitation on its shape and can be used in either powdery or lump shape, or in shaped forms such as granular, cylindrical, spherical, ring, extruded, round-grain, and honeycomb. The shape of the catalyst can be formed by a known method suitable for each shape and the size of the catalyst can be selected arbitrarily in accordance with the size of a reactor. Moreover, to the shaped bodies, known shaping assistants, strength improver or pore making agent can be added if needed.

Pre-contacting gas (G1)

The pre-contacting gas (G1) used in the pre-contacting step of the present invention comprises at least one selected from lower hydrocarbons and a hydrogen gas.

The pre-contacting gas (G1) used in the present invention can be arbitrarily selected from at least one or both of hydrogen and lower hydrocarbons which comprise, as a main component, methane, ethane, propane, butane, ethylene, propylene and butene. The content of the main component in the lower hydrocarbon constituting the pre-contacting gas (G1) is usually not less than 50 % by volume, preferably not less than 70 % by volume, more preferably not less than 90 % by volume, still preferably not less than 99 % by volume, especially not less than 99.9 % by volume. The lower hydrocarbons constituting the pre-contacting gas (G1) may contain components such as nitrogen, helium, argon, oxygen, carbon dioxide and the like in slight amounts incapable of affecting the pre-contacting step. Examples of the pre-contacting gas (G1) are lower hydrocarbons essentially containing methane, lower hydrocarbons essentially containing ethane, lower hydrocarbons essentially containing a mixture of methane and ethane, lower hydrocarbons essentially containing a mixture of methane, ethane and propane, hydrogen, a mixed gas of hydrogen and a lower hydrocarbon essentially containing methane, a mixed gas of hydrogen and a lower hydrocarbon essentially containing ethane, a mixed gas of hydrogen and a lower hydrocarbon essentially containing a mixture of methane and ethane, or a mixed gas of hydrogen and a lower hydrocarbon essentially containing methane, ethane and propane, preferably the mixed gas of hydrogen and a lower hydrocarbon essentially containing methane, more preferably the mixed gas of methane and hydrogen. Moreover, it is possible to use two or more kinds of gas by switching each other during the treatment, for example, two or more kinds of gas selected from methane, hydrogen and a mixed gas of methane and hydrogen, or two or more kinds of methane-hydrogen mixed gas having a different mixed ratio.

In the case of using the mixed gas of hydrogen and a lower hydrocarbon essentially containing methane, or a mixed gas of methane and hydrogen as the pre-contacting gas (G1), the mixed ratio of hydrogen to methane (molar ratio) in the mixed gas is preferably more than 1, more preferably more than 4, furthermore preferably more than 5, especially not less than 10. That is to say, the mixed gas contains, based on 1 mol of methane, hydrogen in an amount of preferably more than 1 mol, more preferably more than 4 mol, furthermore preferably more than 5 mol, especially not less than 10 mol.

Pre-contacting

In the pre-contacting step of the present invention, the above molybdenum-containing solid catalyst is allowed to contact with the pre-contacting gas (G1).

In the present invention, prior to the pre-contacting step, the atmospheric gas in the catalyst layer may be purged with a non-oxidizing inert gas such as nitrogen, helium, argon, etc. The purge of the atmospheric gas in the catalyst layer with this inert gas is preferably carried out at a temperature not higher than 350°C. The purge of the atmospheric gas in the catalyst layer with this inert gas is preferably carried out before the start of the pre-contacting step because the pre-contacting treatment step can be more favorably carried out in a non-oxidizing condition.

In the pre-contacting step of the present invention, the contact of the pre-contacting gas (G1) and the above molybdenum-containing solid catalyst is initiated at a low temperature and thereafter, the temperature of the catalyst layer is preferably increased in the gas stream. Specifically, the contact of the pre-contacting gas (G1) and the catalyst is started at a temperature of preferably not higher than 350°C, more preferably not higher than 250°C. The overall reaction step is desirably carried out in a non-oxidizing condition. Namely, from the start of the pre-contacting step to the start of the reaction step, it is preferred to carry out the procedure in a condition such that the catalyst is not contacted with air or oxygen.

Moreover, after the initiation of the pre-contacting, the pre-contacting temperature (the temperature of the catalyst layer) is increased and held at a holding temperature (Th), which is lower than the reaction temperature (Tr) in the reaction step, and thereafter, the reaction is preferably initiated with changing the feeding gas to the reaction gas.

The holding temperature (Tr) is preferably not lower than 400°C, more preferably not lower than 500°C.

Furthermore, after the start of the pre-contacting treatment, the pre-contacting temperature (the temperature of the catalyst layer) is increased to reach the reaction temperature (Tr) and then held for a certain period of time. Thereafter, the feeding gas is preferably changed to the reaction gas.

Alternatively, after the initiation of the pre-contacting treatment, the pre-contacting temperature (the temperature of the catalyst layer) is increased and reaches the reaction temperature (Tr), and thereafter, the feeding gas is preferably changed to the reaction gas without holding time.

In the pre-contacting step, the pre-contacting gas (G1) is preferably fed at a feeding rate of the certain rate or more, at least the same as the flow rate (feeding rate) of the reaction gas (G2) in the following reaction step, that is to say, based on the flow rate (feeding rate) of the reaction gas (G2) in the reaction step, one time or more, preferably two times or more, more preferably more than 5 times, especially preferably 10 times or more. Alternatively, the ratio (F/W) of the flow rate (F) of the pre-contacting gas (G1) to the catalyst weight (W) is not less than 50 cc/ (g·min), preferably not less than 100 cc/ (g·min), more preferably not less than 200 cc/(g·min).

In the present invention, the pre-contacting step is preferably carried out in a reactor in which the following reaction step is carried out. Alternatively, it is also preferred to carry out the pre-contacting step and the reaction step respectively using reactors connected mutually by moving the catalyst from one reactor to the other reactor. In the present invention, the pre-contacting step and the reaction step are preferably carried out continuously without contacting the pre-contacted molybdenum-containing solid catalyst with air. Moreover, the pre-contacted molybdenum-containing solid catalyst is preferably submitted to the reaction step without cooling because the steps can be carried out economically in good energy efficiency.

Reaction step

Raw material gas (G2)

In the reaction step of the present invention, a lower hydrocarbon gas, which comprises methane as a main component (hereinafter sometimes referred to as raw material gas (G2)) is used as a reaction raw material.

The raw material gas (G2) is a lower hydrocarbon gas which comprises methane as a main component, and contains methane in an amount of usually not less than 50 % by volume, preferably not less than 70 % by volume, more preferably not less than 90 % by volume, furthermore preferably not less than 99 % by volume, especially not less than 99.9 % by volume. The raw material gas (G2) may contain a lower hydrocarbon having 2 to 6 carbon atoms as a component other than methane. Examples thereof are alkanes such as ethane, propane, etc. and alkenes such as ethylene, propylene, etc. Moreover, the raw material gas (G2) may contain nitrogen, helium, argon, oxygen, carbon dioxide, etc. in a slight amount such that they do not affect the reaction.

Methane is contained in natural gas, associated gas, or unconventional natural gas obtainable in petrochemical industries or petroleum refining industries such as off-gas, methane hydrate, coal-bed methane, oil shade gas, biogas, biomass gas, gas obtainable by gasification of biomass, swamp gas and the like. These kinds of gas can be used as they are or after regulating the composition by mixing them with other gas or removing and separating a part of them, as the raw material gas (G2) according to the present invention. Moreover, methane prepared by the reaction of water with carbon monoxide and the like can be used as the raw material gas (G2) in place of the above mentioned kinds of gas.

The raw material gas (G2) preferably does not contain any substances that may cause the activity deterioration of the catalyst. In accordance with necessity, before the reaction step, a process to separate and remove compounds containing nitrogen, sulfur, phosphorus, etc., or a large amount of water, hydrogen, carbon monoxide, carbon dioxide, etc. can be provided to regulate the concentration.

When the raw material gas (G2) for feeding the reaction step contains hydrogen, the yield of the aromatic hydrocarbon is lowered. Therefore, the raw material gas (G2) preferably has a smaller content of a hydrogen gas and desirably has a hydrogen concentration of preferably less than 8 % by volume, more preferably less than 5 % by volume, furthermore preferably less than 4 % by volume, still more preferably less than 2 % by volume, especially less than 1 % by volume, most preferably less than 0.5 % by volume.

Reaction

In the reaction step of the present invention, the molybdenum-containing solid catalyst which has been pre-contacted with the pre-contacting gas (G1) by the above pre-contacting step (hereinafter sometimes referred to as pre-contacted catalyst) is allowed to contact with the raw material gas (G2) which comprises a lower hydrocarbon essentially containing methane to generate the aromatic hydrocarbon.

When the raw material gas (G2) for feeding the reaction step contains hydrogen, the yield of the aromatic hydrocarbon is lowered. Accordingly, in the case of using the gas containing hydrogen, it is preferred to regulate the hydrogen concentration previously before feeding the gas to the inlet of the reactor and to feed the raw material gas (G2) substantially free of hydrogen into the reactor. The expression "substantially free of hydrogen" described above indicates such a fact the influence of decrease of the yield of aromatics caused by the presence of hydrogen is as low as a negligible level. As described above, the raw material gas (G2) for feeding the reactor has a hydrogen concentration of preferably less than 8 %, more preferably less than 5 %, furthermore preferably less than 4 %, still preferably less than 2 %, still more preferably less than 1%, especially less than 0.5%.

The reaction temperature (the temperature of the catalyst layer), which is not particularly limited in the present invention, is preferably 600 to 950°C, more preferably 650 to 800°C, furthermore preferably 700 to 750°C. When the reaction temperature is too low, a high yield cannot be attained by equilibrium constraint, while when the reaction temperature is too high, formation of coke substances, which is a side reaction, is increased, which is unfavorable from the viewpoints of both the yield and the catalyst life.

The reaction can be carried out in any one condition of at ordinary pressure, under pressure and under reduced pressure. The reaction pressure is preferably about from 0.1 to 0.8 MPa, more preferably about from 0.1 to 0.4 MPa, furthermore preferably about from 0.1 to 0.3 MPa, still preferably about 0.1 to 0.2 MPa. In the viewpoint of the efficiency of the reactor, a high pressure is favorable, while in the viewpoint of equilibrium constraint, the reaction is favorably carried out at a low pressure. Needless to add, the present invention should not be limited in the above pressure range.

The reaction can be also carried out in a diluted condition such that an inert gas is separately added into the reaction system in addition to the raw material gas (G2). Examples of the inert gas may include nitrogen, helium, argon and the like.

As the type of the reactor, it is possible to employ an arbitrary type such as a fixed bed type, a fluidized bed type, a moving bed type, a transport bed type, a circulating fluidized bed type or their combination bed types. For example, in the fixed bed, any one of a packed-bed type, a radial flow type, a monolith type and the like can be arbitrarily selected and the direction of the gas flow is not particularly limited. Moreover, in the fluidized bed, the moving bed or the transport bed, the moving rate of the catalyst particles and the flow rate of the gas can be arbitrarily selected in the range that the reaction system can be conducted respectively.

As the method of heat transfer of the reactor, it is possible to employ any one of a heat insulating method and a heat exchanging method. Because the reaction is a endothermic reaction absorbing large amount of heat, it is preferred to employ ,in the heat insulating method, a multi-stage reactor system with heating layers between the reactors. In the heat exchanging method, it is possible to employ an electric heating method, a heat medium heating method or a direct heating method. Particularly, it is preferred to employ the direct heating method capable of carrying out the reaction at a high temperature. On the same reasons as above, it is preferred to compensate the temperature drop induced by the reaction by dividing the heating zone into multiple zones and controlling them.

Regarding the reactor, only one reactor can be used, or alternatively, two or more reactors connected in series can be used in such a way that an outlet gas of one reactor is fed to the next reactor so that the conversion can be enhanced. In the case of employing two or more reactors, between the two reactors, other gas may be mixed or a part of the gas may be separated and removed for regulating the composition or heating the gas again.

After the reaction, the reaction products can be separated and recovered by a known separation method. Unreacted lower hydrocarbons such as methane and the like can be separated by a known method and recycled. Moreover, hydrogen coproduced by the reaction, unreacted methane and the like can be separated and recovered by a known method and can be utilized as a raw material or an energy source for the other reactors.

Example

The present invention will be described in more detail with reference to the following examples, but it should not be limited by these examples.

Reaction method

For the catalyst evaluation, a fixed bed flow type reactor was used.

In the following Examples and Comparative Examples, unless otherwise stated, pure methane (supplied by JFP) was used as a reaction gas (raw material gas (G2)) and to the pure methane, high-purity nitrogen was mixed in an amount of 10% of methane and fed as an internal standard for analysis. Moreover, unless otherwise stated, the feeding rate of methane was 7.5 cc/min.

As a reaction tube, a double tube having a quartz inner tube (inner diameter 7 mm) and a Hastelloy C276 outer tube was used. Unless otherwise stated, the reaction was carried out at 700°C at atmospheric pressure.

Analysis and Evaluation methods

An outlet gas from a reactor was directly analyzed by an on-line gas chromatograph (GC2014, GC14A or GC8A, manufactured by Shimadzu Co.).

With regard to the concentration of each component in a generated gas stream, the concentration of an inorganic gas component was determined with reference to the internal standard nitrogen and that of an organic substance was determined with reference to methane which is quantified by the above-mentioned method referenced to nitrogen.

The yield of benzene was defined by the following formula 1, and the activity preservation ratio was defined by the following formula 2, respectively. A catalyst is judged excellent when both of the indices are high.

```
Formula 1

Yield of benzene (%)

= 100 x Amount of benzene generated (mol) x 6 / Amount of methane

fed (mol)

Formula 2

Activity preservation ratio (%)

= 100 x (Yield of benzene (%) at 18.5 hr after the start of the

reaction) / (Maximum value of yield of benzene (%))
```

Catalyst Preparation Example 1

Preparation of Mo/ZSM-5 catalyst (Catalyst A)

Ammonium heptamolybdate ($(NH_4)_6Mo_7O_{24} \cdot 4H_2O$, Special grade, purchased from Wako Pure Chemical Industries, Ltd.) was dissolved in ion-exchanged water. The amount of a molybdenum salt used was determined by such a calculation that the amount of molybdenum supported was 12 % by weight based on a catalyst after the preparation. To this solution, 5.0 g of ammonium type ZSM-5 zeolite (CBV3024E having a ratio of silica to alumina of 30, manufactured by Zeolyst International) was suspended and stirred for a while. Thereafter, the mixture was dried at 120°C and calcined at 500°C, to prepare a catalyst. The catalyst was referred as Catalyst A.

Example 1

A reactor was charged with 0.3 g of the Catalyst A prepared in the Catalyst Preparation Example 1, and was purged with helium. Thereafter, the temperature was increased to 200°C in a helium stream (10 cc/min) and maintained for 30 min. Subsequently, the gas for supply to the reaction tube was changed to a pre-contacting gas, which was a mixed gas of methane (7.5 cc/min) and hydrogen (12 cc/min) and the temperature was increased to 600°C. The temperature was held for 90 min and thereby a pre-contacting was completed. After completion of the pre-contacting, the mixed gas was changed to the reaction gas, the temperature was increased to 700°C and held, and thereby the reaction was carried out. The maximum yield of benzene was 6. 9 % and the activity preservation ratio was 79 %.

Example 2

The reaction was carried out in the same manner as in Example 1 except that the holding at 600 °C in the pre-contacting step was not carried out and therefore the holding time was 0. The maximum yield of benzene was 6.3 % and the activity preservation ratio was 63%.

Example 3

The reaction was carried out in the same manner as in Example 1 except that the holding time at 600°C in the pre-contacting step was 30 min. The maximum yield of benzene was 7.0 % and the activity preservation ratio was 81 %.

Example 4

The reaction was carried out in the same manner as in Example 1 except that the holding time at 600°C in the pre-contacting step was 180 min. The maximum yield of benzene was 7.0 % and the activity preservation ratio was 78 %.

Comparative Example 1

A reactor was charged with the Catalyst A and was purged with helium in the same manner as in Example 1, and then the temperature was increased to 700°C in a helium stream (10 cc/min) and held for 40 min. Subsequently, the gas was changed to the reaction gas and the reaction was started. The maximum yield of benzene was 3.7 % and the activity preservation ratio was 6 %.

Examples 5 to 7

In each Example, the reaction was carried out in the same manner as in Example 1 except that the flow rates of hydrogen in the mixed gas used as the pre-contacting gas were 1.5 cc/min, 30 cc/min and 75 cc/min respectively. The maximum yields of benzene were 4.7 %, 6.8 % and 7.2 % respectively, and the activity preservation ratios were 29 %, 89 % and 91 % respectively.

Example 8

A reactor was charged with the Catalyst A, and was purged with helium in the same manner as in Example 1. Thereafter, the temperature was increased to 200°C in a helium stream (10 cc/min) and held for 30 min. Subsequently, the gas was changed to a pre-contacting gas, which was a mixed gas of methane (7.5 cc/min) and hydrogen (75 cc/min) and the temperature was increased to 700°C. The temperature was held for 80 min and thereby the pre-contacting step was completed. After completion of the pre-contacting step, the mixed gas was changed to the reaction gas and the reaction was initiated. The maximum yield of benzene was 7.3 % and the activity preservation ratio was 93 %.

Examples 9 to 11

In each Example, the reaction was carried out in the same manner as in Example 8 except for using, as the pre-contacting gas, a mixed gas of methane (7.5 cc/min) and hydrogen (12 cc/min), a mixed gas of methane (7.5 cc/min) and hydrogen (100 cc/min) and only hydrogen (100 cc/min), respectively. The maximum yields of benzene were 6.4 %, 7.2 % and 6.9 % respectively, and the activity preservation ratios were 80 %, 95 % and 94 % respectively.

Examples 12 and 13

In each Example, the reaction was carried out in the same manner as in Example 8 except that the holding temperatures in a helium stream were changed to 350°C and 30°C, respectively. The maximum yields of benzene were 7.1 % and 7.0 %, respectively, and the activity preservation ratios were 92 % and 94 % respectively.

Example 14

The reaction was carried out in the same manner as in Example 8 except that hydrogen (0.39 cc/min) was added to the reaction gas. The maximum yield of benzene was 5.6 % and the activity preservation ratio was 92 %.

Example 15

The reaction was carried out in the same manner as in Example 8 except that the holding time at 700°C in the contacting step with the pre-contacting gas was 0 min, and the gas was changed to the reaction gas at the same time that the temperature reached 700°C. The maximum yield of benzene was 7.2 % and the activity preservation ratio was 91 %.

Example 16

The reaction was carried out in the same manner as in Example 1 except that the flow rate of hydrogen in the pre-contacting gas was 75 cc/min, and the holding temperature was changed from 600°C to 650°C. The maximum yield of benzene was 7.0 % and the activity preservation ratio was 92 %.

Examples 17 to 20

In each Example, a reactor was charged with 0.3 g of the Catalyst A, and was purged with helium in the same manner as in Example 1. Thereafter, the temperature was increased to 200°C in a helium stream (10 cc/min) and held for 30 min. Subsequently, the gas was changed to a pre-contacting gas, which was a mixed gas of methane (7.5 cc/min) and hydrogen (75 cc/min) and the temperature was increased as shown in Table 1. The temperature was held for 80 min and thereby the pre-contacting step was completed. After completion of the pre-contacting, the mixed gas was changed to the reaction gas, the temperature was increased to 700°C and the reaction was initiated. The maximum yields of benzene and the activity preservation ratios are shown in Table 1.

Table 1

| Example | Temperature increased and held (°C) in the pre-contacting | Maximum yield of Benzene (%) | Activity preservation ratio (%) |
|---|---|---|---|
| 17 | 600 | 7.1 | 90 |
| 18 | 500 | 7.1 | 90 |
| 19 | 400 | 5.3 | 61 |
| 20 | 300 | 3.5 | 17 |

Examples 21 to 23

In each Example, a reactor was charged with 0.3 g of the Catalyst A, and was purged with helium in the same manner as in Example 1. Thereafter, the temperature was increased to 200°C in a helium stream (10 cc/min) and held for 30 min. Subsequently, the gas was changed to a pre-contacting gas, which was a mixed gas of methane (7.5 cc/min) and hydrogen (30 cc/min) and the temperature was increased as shown in Table 2. The temperature was held for 80 min and thereby the pre-contacting was completed. After completion of the pre-contacting, the mixed gas was changed to the reaction gas, and the reaction was initiated. The maximum yields of benzene and the activity preservation ratios are shown in Table 2.

Table 2

| Example | Temperature increased and held (°C) in the pre-contacting | Maximum yield of Benzene (%) | Activity preservation ratio (%) |
|---|---|---|---|
| 21 | 700 | 6.8 | 88 |
| 22 | 500 | 7.0 | 88 |
| 23 | 300 | 3.7 | 18 |

Example 24

The reaction was carried out in the same manner as in Example 20 except that helium was changed to nitrogen. The maximum yield of benzene was 7.0 % and the activity preservation ratio was 90 %.

Catalyst Preparation Examples 2 to 8

Preparation of Mo/ZSM-5 catalyst (Catalysts B to H)

In each Example, the catalyst was prepared in the same manner as in Catalyst Preparation Example 1 except that the amounts of molybdenum supported were 3, 5, 6, 8, 10, 15 and 20 % by weight respectively. The resulting catalysts were referred as Catalysts B, C, D, E, F, G and H, respectively.

Catalyst Preparation Example 9

Preparation of Mo/ZSM-5 catalyst (Catalyst I)

Ammonium heptamolybdate ($(NH_4)_6Mo_7O_{24} \cdot 4H_2O$, Special grade, purchased from Wako Pure Chemical Industries, Ltd.) and 5.0 g of ammonium type ZSM-5 zeolite (CBV3024E having a ratio of silica to alumina of 30, manufactured by Zeolyst International) were mixed. The amount of a molybdenum salt used was determined by such a calculation that the content of molybdenum was 15 % by weight based on a catalyst after the preparation. The resulting powder was dried and calcined in the same manner in Catalyst Preparation Example 1, to prepare a catalyst. The catalyst was referred as Catalyst I.

Catalyst Preparation Examples 10 to 12

Preparation of Mo/ZSM-5 catalysts (Catalyst J, K and L)

In each Example, a catalyst was prepared in the same manner in Catalyst Preparation Example 9 except that molybdenum trioxide ($MoO_3$, purchased from Wako Pure Chemical Industries, Ltd.) was used in place of ammonium heptamolybdate and the amounts of molybdenum supported were 5, 10 and 15 % by weight respectively. The resulting catalysts were referred as Catalysts J, K and L, respectively.

Examples 25 to 34

In each Example, a reaction was carried out in the same manner as in Example 1 except for using the Catalysts B, C, E, F, G, H, I, J, K and L respectively. The maximum yields of benzene and the activity preservation ratios are shown in Table 3.

Table 3

| Example | Catalyst | Maximum yield of Benzene (%) | Activity preservation ratio (%) |
|---|---|---|---|
| 25 | B | 6.1 | 66 |
| 26 | C | 6.3 | 81 |
| 27 | E | 6.9 | 81 |
| 28 | F | 6.8 | 81 |
| 29 | G | 6.7 | 73 |
| 30 | H | 6.2 | 67 |
| 31 | I | 6.0 | 62 |
| 32 | J | 6.3 | 81 |
| 33 | K | 6.5 | 81 |
| 34 | L | 6.4 | 69 |

Examples 35 and 36

In each Example, a reaction was carried out in the same manner as in Example 8 except for using the Catalysts C and D respectively in place of the Catalyst A. The maximum yields of benzene were 6.7% and 7.1% respectively and the activity preservation ratios were 87% and 94% respectively.

Comparative Examples 2 to 9

In each Example, a reaction was carried out in the same manner as in Comparative Example 1 except for using the Catalysts B, C, D, E, F, G, J and K respectively in place of the Catalyst A. The maximum yields of benzene and the activity preservation ratios are shown in Table 4.

Table 4

| Comparative Example | Catalyst | Maximum yield of Benzene (%) | Activity preservation ratio (%) |
|---|---|---|---|
| 2 | B | 5.5 | 53 |
| 3 | C | 6.3 | 50 |
| 4 | D | 4.5 | 10 |
| 5 | E | 4.5 | 12 |
| 6 | F | 4.4 | 12 |
| 7 | G | 3.0 | 4 |
| 8 | J | 6.1 | 55 |
| 9 | K | 4.9 | 21 |

Example 37

A reactor was charged with 0.3 g of the Catalyst A, and the temperature was increased to 600°C in an air stream (10 cc/min) and held for 30 min. Subsequently, the gas was changed to a pre-contacting gas, which was a mixed gas of methane (7.5 cc/min) and hydrogen (30 cc/min) and the temperature was held at 600°C for 90 min. Thereafter, the mixed gas was changed to the reaction gas, the temperature was increased and the reaction was initiated. The maximum yield of benzene was 6.3 % and the activity preservation ratio was 73 %.

Comparative Examples 10 and 11

In each Example, a reactor was charged with 0.3 g of the Catalyst C or J, and the temperature was increased to 700°C in an air stream (10 cc/min) and held for 40 min. Subsequently, purge was carried out with passing helium (100 cc/min) for 5 min while keeping the temperature. Thereafter, the gas was changed to the reaction gas and the reaction was initiated. The maximum yields of benzene were 5.2 % and 4.8% respectively, and the activity preservation ratios were 40 % and 33% respectively.

Catalyst Preparation Comparative Examples 1 to 4

Preparation of Mo/ZSM-5 catalysts (Catalyst M to P)

In each Example, a catalyst was prepared in the same manner as in Catalyst Preparation Example 1 except that the amounts of molybdenum supported were 3, 5, 8 and 12 % by weight respectively, and ammonium type ZSM-5 zeolite (CBV5524G having a ratio of silica to alumina of 50, manufactured by Zeolyst International) was used. The resulting catalysts were referred as Catalysts M, N, O and P respectively.

Catalyst Preparation Comparative Examples 5 to 8

Preparation of Mo/ZSM-5 catalysts (Catalyst Q to T)

In each Example, a catalyst was prepared in the same manner in Catalyst Preparation Example 1 except that the amounts of molybdenum supported were 3, 5, 8 and 12 % by weight respectively, and ammonium type ZSM-5 zeolite (CBV8014 having a ratio of silica to alumina of 80, manufactured by Zeolyst International) was used. The resulting catalysts were referred as Catalysts Q, R, S and T respectively.

Comparative Example 12

A reaction was carried out in the same manner as in Example 8 except for using the Catalyst N in place of the Catalyst A. The maximum yield of benzene was 6.5 % and the activity preservation ratio was 88 %.

Comparative Examples 13 to 20

In each Example, a reaction was carried out in the same manner as in Example 1 except for using the Catalysts M, N, O, P, Q, R, S and T respectively in place of the Catalyst A. The maximum yields of benzene and the activity preservation ratios are shown in Table 5.

Table 5

| Comparative Example | Catalyst | Maximum yield of Benzene (%) | Activity preservation ratio |
|---|---|---|---|
| 13 | M | 5.0 | 59 |
| 14 | N | 5.9 | 76 |
| 15 | O | 6.4 | 64 |
| 16 | P | 6.2 | 60 |
| 17 | Q | 4.4 | 29 |
| 18 | R | 4.5 | 32 |
| 19 | S | 4.7 | 33 |
| 20 | T | 4.7 | 22 |

Comparative Examples 21 to 28

In each Example, a reaction was carried out in the same manner as in Comparative Example 1 except for using the Catalysts M, N, O, P, Q, R, S and T respectively in place of the Catalyst A. The maximum yields of benzene and the activity preservation ratios are shown in Table 6.

Table 6

| Comparative Example | Catalyst | Maximum yield of Benzene (%) | Activity preservation ratio (%) |
|---|---|---|---|
| 21 | M | 4.2 | 32 |
| 22 | N | 5.2 | 37 |
| 23 | O | 3.3 | 6 |
| 24 | P | 3.2 | 5 |
| 25 | Q | 2.1 | 22 |
| 26 | R | 2.3 | 19 |
| 27 | S | 2.5 | 6 |
| 28 | T | 2.5 | 6 |

Comparative Example 29

A reactor was charged with 0.3 g of the Catalyst N, and was purged with helium and the temperature was increased to 200°C in a helium stream (10 cc/min) and held for 30 min. Thereafter, the temperature was increased to 700°C and held for 40 min. Subsequently, the gas was changed to the reaction gas and the reaction was initiated. The maximum yield of benzene was 4.7 % and the activity preservation ratio was 26 %.

Examples 38 to 39 and Comparative Example 30

In each Example, a reactor was charged with 0.3 g of the Catalyst A or N, the inside of the tube was purged with helium and the temperature was increased to 200°C in a helium stream (10 cc/min) and held for 30 min. Subsequently, the gas was changed to a pre-contacting gas of methane (7.5 cc/min) or hydrogen (12 cc/min) and the temperature was increased to 600°C. The temperature was held for 90 min, the gas was changed to the reaction gas and the reaction was initiated. The results are shown in Table 7.

Table 7

| Example | Catalyst | Gas kind in Pre-contacting | Maximum yield of Benzene (%) | Activity preservation ratio (%) |
|---|---|---|---|---|
| 38 | A | Methane | 6.4 | 60 |
| 39 | A | Hydrogen | 5.6 | 52 |
| Comp. Ex. 30 | N | Methane | 4.9 | 53 |

Examples 40 and 41

In each Example, a reactor was charged with 0.3 g of the Catalyst A, and was purged with helium and the temperature was increased to 200°C in a helium stream (10 cc/min) and held for 30 min. Subsequently, the gas was changed to a pre-contacting gas of methane (55 cc/min) and the temperature was increased as shown in Table 8. The temperature was held for the time as shown in Table 8, to perform a pre-contacting. Thereafter, the gas was changed to the reaction gas and the reaction was initiated. The maximum yields of benzene and the activity preservation ratios are shown in Table 8.

Table 8

| Example | Pre-contacting gas | | Maximum yield of Benzene (%) | Activity preservation ratio (%) |
|---|---|---|---|---|
| | Temperature (°C) | Time (min) | | |
| 40 | 700 | 80 | 6.7 | 56 |
| 41 | 600 | 90 | 7.2 | 87 |

Examples 42 to 45

In each Example, a reactor was charged with 0.3 g of the Catalyst A, and was purged with helium and the temperature was increased to 200 ° C in a helium stream (10 cc/min) and held for 30 min. Subsequently, the gas was changed to a pre-contacting gas, which was a mixed gas of methane and hydrogen (the flow rates as shown in Table 9) and then the temperature was increased to 700 ° C and was held for 80 min. Next, only the hydrogen feeding was stopped and methane was fed at the same flow rate as the reaction gas and then the reaction was initiated. The results are shown in Table 9.

Table 9

| Example | Pre-contacting gas | | Maximum yield of Benzene (%) | Activity preservation ratio (%) |
|---|---|---|---|---|
| | Methane flow rate (cc/min) | Hydrogen flow rate (cc/min) | | |
| 42 | 2.0 | 75 | 7.4 | 100 |
| 43 | 3.8 | 75 | 7.2 | 100 |
| 44 | 15.0 | 75 | 6.8 | 69 |
| 45 | 3.8 | 38 | 7.1 | 99 |

Catalyst Preparation Comparative Example 9

Preparation of Mo/ZSM-5 catalyst (Catalyst U)

Ammonium heptamolybdate ($(NH_4)_6Mo_7O_{24} \cdot 4H_2O$, Special grade, purchased from Wako Pure Chemical Industries, Ltd.) was dissolved in ion-exchanged water. The amount of a molybdenum salt used was determined by such a calculation that the amount of molybdenum supported was 5 % by weight based on a catalyst after the preparation. To the solution, 5.0 g of ammonium type ZSM-5 zeolite (CBV5524G having a ratio of silica to alumina of 50, manufactured by Zeolyst International) was dissolved and stirred for a while. Thereafter, the mixture was dried at 120 ° C and calcined at 500 °C, to prepare a molybdenum-supported zeolite powder.

Furthermore, in ion-exchanged water, lanthanum nitrate ($La(NO_3)_3 \cdot 6H_2O$, purchased from Wako Pure Chemical Industries, Ltd.) was dissolved in an amount such that the amount of lanthanum supported in a resulting catalyst was 3 % by weight, to prepare a solution. To the solution, the molybdenum-supported zeolite powder was suspended and stirred for a while. Thereafter, the suspension was dried at 120°C and calcined at 500°C to prepare a catalyst. The catalyst was referred as Catalyst U.

Catalyst Preparation Comparative Examples 10 and 11

Preparation of Mo/ZSM-5 catalyst (Catalysts V and W)

In each Example, the procedure of Catalyst Preparation Comparative Example 9 was repeated except for using, in place of lanthanum nitrate, samarium nitrate ($Sm(NO_3)_3 \cdot 6H_2O$, purchased from Wako Pure Chemical Industries, Ltd.) in an amount such that the amount of samarium supported in a resulting catalyst was 3 % by weight and cobalt nitrate ($Co(NO_3)_3 \cdot 6H_2O$, purchased from Wako Pure Chemical Industries, Ltd.) in an amount such that the amount of cobalt supported in a resulting catalyst was 2 % by weight respectively. The resulting catalysts were referred as Catalyst V and Catalyst W respectively.

Catalyst Preparation Comparative Example 12

Preparation of Mo/ZSM-5 catalyst (Catalyst X)

Ammonium heptamolybdate (($NH_4$)$6Mo_7O_{24} \cdot 4H_2O$, Special grade, purchased from Wako Pure Chemical Industries, Ltd.) in an amount such that the amount of molybdenum supported in a resulting catalyst was 5 % by weight and ammonium metatungstate ($NH_4$)$_6$-[$H_2W_{12}O_{40}$], purchased from Aldrich Co.) in an amount such that the amount of tungsten supported in the resulting catalyst was 1 % by weight were dissolved in ion-exchanged water to prepare a solution. To the solution, 5. 0 g of ammonium type ZSM-5 zeolite (CBV5524G having a ratio of silica to alumina of 50, manufactured by Zeolyst International) was suspended and stirred for a while. Thereafter, the suspension was dried at 120°C and calcined at 500°C, to prepare a catalyst. The catalyst was referred as Catalyst X.

Comparative Example 31

A reactor was charged with 0.3 g of the Catalyst U, and was purged with helium and the temperature was increased to 200°C in a helium stream (10 cc/min) and held for 30 min. Subsequently, the gas was changed to a pre-contacting gas, which was a mixed gas of methane (7.5 cc/min) and hydrogen (30 cc/min) and the temperature was increased to 600°C. The temperature increased was held for 90 min. Thereafter, the gas was changed to the reaction gas and the reaction was initiated. The maximum yield of benzene was 4.4 % and the activity preservation ratio was 24 %.

Comparative Example 32

The procedure of Comparative Example 31 was repeated except for using the Catalyst V in place of the Catalyst U to carry out a reaction. The maximum yield of benzene was 4.1 % and the activity preservation ratio was 27 %.

Comparative Examples 33 and 34

In each Example, the procedure of Comparative Example 31 was repeated except for using a mixed gas of methane (7.5 cc/min) and hydrogen (75 cc/min) as a pre-contacting gas and using the Catalysts W and X respectively in place of the Catalyst U to carry out a reaction. The maximum yields of benzene of the catalysts were 4.5 % and 6.3% respectively and the activity preservation ratios of the catalysts were 71 % and 79% respectively.

Comparative Examples 35 to 38

In each Example, the procedure of Comparative Example 1 was repeated except for using the catalysts U, V, W and X respectively in place of the catalyst A to carry out a reaction. The maximum yields of benzene of the catalysts were 3.2 %, 3.5%, 2.3% and 4.1% respectively and the activity preservation ratios of the catalysts were 11 %, 12%, 15% and 24% respectively.

Catalyst Preparation Comparative Examples 13 and 14

Preparation of Mo/ZSM-5 catalysts (Catalysts Y and Z)

In each Example, the procedure of Catalyst Preparation Comparative Example 12 was repeated except for using aluminum nitrate (Al(NO$_3$)$_3$ · 9H$_2$O, purchased from Wako Pure Chemical Industries, Ltd.) in an amount such that the amount of aluminum supported in a resulting catalyst was 0.5 % by weight or gallium nitrate (Ga(NO$_3$)$_3$ · nH$_2$O, purchased from Wako Pure Chemical Industries, Ltd.) in an amount such that the amount of gallium supported in a resulting catalyst was 1% by weight in place of ammonium metatungstate to prepare a catalyst. The resulting catalysts were referred as Catalysts Y and Z.

Catalyst Preparation Example 13

Preparation of Mo/ZSM-5 catalyst (Catalyst AA)

Ammonium heptamolybdate ((NH$_4$)$_6$Mo$_7$O$_{24}$· 4H$_2$O, Special grade, purchased from Wako Pure Chemical Industries, Ltd.) was dissolved in ion-exchanged water. The amount of a molybdenum salt used was determined by such a calculation that the amount of molybdenum supported was 5% by weight based on a catalyst after the preparation. To the solution, 5.0 g of ammonium type ZSM-5 zeolite (CBV3024E having a ratio of silica to alumina of 30, manufactured by Zeolyst International) was suspended and stirred for a while. Thereafter, the suspension was dried at 120°C and calcined at 500°C, to prepare a molybdenum-supported zeolite powder.
Furthermore, in ion-exchanged water, silver nitrate (AgNO$_3$, purchased from Wako Pure Chemical Industries, Ltd.) was dissolved in an amount such that the amount of silver supported in a resulting catalyst was 2% by weight, to prepare a solution. To the solution, the molybdenum-supported zeolite powder was suspended and stirred for a while. Thereafter, the suspension was dried at 120°C and calcined at 500°C to prepare a catalyst. The catalyst was referred as Catalyst AA.

Comparative Examples 39 and 40, and Example 46

In each Example, a reactor was charged with 0.3 g of the Catalysts Y, Z or AA, and was purged with helium and the temperature was increased to 200°C in a helium stream (10 cc/min) and held for 30 min. Subsequently, the gas was changed to a pre-contacting gas, which was a mixed gas of methane (7.5 cc/min) and hydrogen (75 cc/min) and the temperature was increased to 700°C. Then, the gas was changed to the reaction gas and the reaction was initiated. The maximum yields of benzene of the catalysts were 5.1 %, 4.9% and 5.7% respectively and the activity preservation ratios of the catalysts were 70 %, 63% and 75% respectively.

Comparative Examples 41 to 43

In each Example, the procedure of Comparative Example 1 was repeated except for using the Catalysts Y, Z or AA in place of the Catalyst A. The maximum yields of benzene of the catalysts were 4.2 %, 4.6% and 4.9% respectively and the activity preservation ratios of the catalysts were 39 %, 17% and 19% respectively.

Catalyst Preparation Examples 14 to 17

Preparation of Mo/ZSM-5 catalysts (Catalyst BB, CC, DD and EE)

In each Example, the procedure of Catalyst Preparation Example 13 was repeated except for using, in place of silver nitrate, yttrium nitrate (Y(NO$_3$)$_3$ · 6H$_2$O, purchased from Sigma-Aldrich Co.) in an amount such that the amount of yttrium supported in a resulting catalyst was 2% by weight, zirconium oxynitrate (ZrO(NO$_3$)$_2$ · 2H$_2$O, purchased from Kanto Chemical Co., Inc.) in an amount such that the amount of zirconium supported in the resulting catalyst was 2 % by weight, copper nitrate (Cu(NO$_3$)$_2$ · 3H$_2$O, purchased from Alfa Aesar) in an amount such that the amount of copper supported in the resulting catalyst was 1 % by weight, or zinc nitrate (Zn(NO$_3$)$_2$ · 6H$_2$O, purchased from Kanto Chemical Co., Inc.) in an amount such that the amount of zinc supported in the resulting catalyst was 1 % by weight, to prepare a catalyst. The resulting catalysts were referred as Catalysts BB, CC, DD and EE respectively.

Catalyst Preparation Comparative Example 15

Preparation of Mo/ZSM-5 catalyst (Catalyst FF)

The procedure of Catalyst Preparation Comparative Example 9 was repeated except for using iron nitrate (Fe(NO$_3$)$_3$ · 9H$_2$O, purchased from Wako Pure Chemical Industries, Ltd.) in an amount such that the amount of iron supported in a resulting catalyst was 0.5 % by weight, in place of lanthanum nitrate, to prepare a catalyst. The resulting catalyst was referred as Catalyst FF.

Catalyst Preparation Comparative Example 16

Preparation of Mo/ZSM-5 catalyst (Catalyst GG)

The procedure of Catalyst Preparation Comparative Example 12 was repeated except for using nickel nitrate (Ni (NO$_3$)$_2$ · 6H$_2$O, purchased from Wako Pure Chemical Industries, Ltd.) in an amount such that the amount of nickel supported in a resulting catalyst was 0.5% by weight, in place of ammonium metatungstate, to prepare a catalyst. The resulting catalyst was referred as Catalyst GG.

Examples 47 to 50 and Comparative Examples 44 and 45

In each Example, a reactor was charged with 0.3 g of the Catalysts BB, CC, DD, EE, FF or GG, and was purged with helium and the temperature was increased to 200 °C in a helium stream (10 cc/min) and held for 30 min. Subsequently, the gas was changed to a pre-contacting gas, which was a mixed gas of methane (7.5 cc/min) and hydrogen (75 cc/min), and the temperature was increased to 700°C and held for 80 min and thereby the pre-contacting step was completed. Then, the gas was changed to a reaction gas and the reaction was initiated. The results are shown in Table 10.

Table 10

| Example | Catalyst | Maximum yield of Benzene (%) | Activity preservation ratio (%) |
|---|---|---|---|
| 47 | BB | 4.6 | 44 |
| 48 | CC | 6.0 | 81 |
| 49 | DD | 6.1 | 83 |
| 50 | EE | 7.0 | 92 |
| Comp. Ex. 44 | FF | 5.9 | 92 |
| Comp. Ex. 45 | GG | 4.4 | 69 |

Comparative Examples 46 to 51

In each Example, the procedure of Comparative Example 1 was repeated except for using the Catalysts BB, CC, DD, EE, FF or GG in place of the Catalyst A to carry out a reaction. The results are shown in Table 11.

Table 11

| Comparative Example | Catalyst | Maximum yield of Benzene (%) | Activity preservation ratio (%) |
|---|---|---|---|
| 46 | BB | 5.0 | 32 |
| 47 | CC | 5.7 | 42 |
| 48 | DD | 3.7 | 18 |
| 49 | EE | 5.4 | 38 |
| 50 | FF | 3.7 | 41 |
| 51 | GG | 3.5 | 31 |

Catalyst Preparation Examples 18 to 21

Preparation of Mo/ZSM-5 catalysts (Catalysts HH, II, JJ and KK)

In each Example, the procedure of Catalyst Preparation Example 13 was repeated except that the amount of molybdenum supported was 6 % by weight and in place of silver nitrate, ruthenium chloride ($RuCl_3 \cdot nH_2O$, purchased from Alfa Aesar), rhodium chloride ($RuCl_3 \cdot nH_2O$, purchased from Alfa Aesar), paladium nitrate ($Pd(NO_3)_2 \cdot nH_2O$, purchased from Strem Chemicals, Inc.) or tetraamineplatinum nitrate ($[Pt(NH_3)_4] (NO_3)_2$, purchased from Aldrich Co.) in an amount such that the molar ratio of metal to molybdenum was 0.2, to prepare a catalyst. The resulting catalysts were referred as Catalysts HH, II, JJ and KK respectively.

Examples 51 to 54

In each Example, the procedure of Example 47 was repeated except for using the Catalysts HH, II, JJ or KK, in place of the Catalysts BB. The results are shown in Table 12.

Table 12

| Example | Catalyst | Maximum yield of Benzene (%) | Activity preservation ratio (%) |
|---------|----------|------------------------------|----------------------------------|
| 51 | HH | 7.1 | 94 |
| 52 | II | 4.5 | 60 |
| 53 | JJ | 5.2 | 74 |
| 54 | KK | 5.8 | 82 |

Comparative Examples 52 to 55

In each Example, the procedure of Comparative Example 1 was repeated except for using the Catalysts HH, II, JJ or KK, in place of the catalyst A. The results are shown in Table 13.

Table 13

| Comparative Example | Catalyst | Maximum yield of Benzene (%) | Activity preservation ratio (%) |
|---------------------|----------|------------------------------|----------------------------------|
| 52 | HH | 5.5 | 35 |
| 53 | II | 4.6 | 27 |
| 54 | JJ | 4.5 | 23 |
| 55 | KK | 4.8 | 21 |

Catalyst Preparation Example 22

Synthesis of Catalyst Support

A catalyst support was synthesized in accordance with a method as described in a literature (K. Okumura, et al., Journal of Catalysis, Vol. 206, P.23 (2002)) in the following manner. An autoclave made of Teflon™ was charged with ST-40 and hexamethyleneimine and subjected to hydrothermal synthesis at 150°C. The resulting solid was dried at 120°C and calcined at 540°C for 6 hr. The resulting powder was subjected to ion-exchange with an aqueous solution (2M) of ammonium nitrate ($NH_4NO_3$, purchased from Wako Pure Chemical Industries, Ltd.) and then calcined in the same manner. It was found by XRD analysis that the resulting solid had a pure MWW structure and by ICP analysis that the ratio of silica to alumina was 23.

Preparation of Catalyst

The procedure of Catalyst Preparation 1 was repeated except for using the powder prepared in the above Synthesis of Catalyst Support in place of ZSM-5 zeolite, to prepare a catalyst. The catalyst was referred as Catalyst LL.

Example 55

The procedure of Example 1 was repeated except for using the Catalyst LL in place of the Catalyst A, to carry out a reaction. The maximum yield of benzene was 8.2 % and the activity preservation ratio was 68 %.

Comparative Example 56

The procedure of Comparative Example 1 was repeated except for using the Catalyst LL in place of the Catalyst A, to carry out a reaction. The maximum yield of benzene was 0.3 % and the activity preservation ratio was 0%.

Example 56

A reactor was charged with 0.3 g of the Catalyst A, and was purged with helium and the temperature was increased to 200°C in a helium stream (10 cc/min) and held for 30 min. Subsequently, the gas was changed to a pre-contacting gas which was ethane (15.7 cc/min), and the temperature was increased to 600°C and held for 90 min and thereby the pre-contacting step was completed. Then, the gas was changed to the reaction gas, the temperature was increased to 700°C and then the reaction was initiated. The maximum yield of benzene was 6.5 % and the activity preservation ratio was 62 %.

Example 57

The procedure of Example 56 was repeated except for changing the pre-contacting gas to a mixed gas of ethane (6.0 cc/min) and hydrogen (30 cc/min) to carry out a reaction. The maximum yield of benzene was 7.4 % and the activity preservation ratio was 86 %.

Example 58

The procedure of Example 56 was repeated except for changing the pre-contacting gas to a mixed gas of methane (7.5 cc/min) and ethane (0.75 cc/min) to carry out a reaction. The maximum yield of benzene was 5.9% and the activity preservation ratio was 56 %.

Examples 59 and 60

In each Example, the procedure of Example 56 was repeated except for changing the pre-contacting gas to a mixed gas of methane (7.5 cc/min), ethane (0.79 cc/min) and hydrogen (30 cc/min) or a mixed gas of methane (7.5 cc/min), ethane (0.75 cc/min) and hydrogen (75 cc/min) to carry out reactions. The maximum yields of benzene of the reactions were 7.1 % and 6.9 % respectively and the activity preservation ratios of the catalysts were 86 % and 87 % respectively.

Example 61

A reactor was charged with 0.3 g of the catalyst A, and was purged with helium and the temperature was increased to 200°C in a helium stream (10 cc/min) and held for 30 min. Subsequently, the gas was changed to a pre-contacting gas, which was a mixed gas of methane (7.1 cc/min), ethane (0.38 cc/min) and hydrogen (75 cc/min) and the temperature was increased to 700°C and held for 80 min. Then, only the hydrogen feeding was stopped, and a reaction was initiated using methane and ethane as a reaction gas at the same flow rates. The maximum yield of benzene was 9.3 % and the activity preservation ratio was 96 %.

Example 62

The procedure of Example 61 was repeated except for changing the pre-contacting gas to a mixed gas of methane (6.75 cc/min), ethane (0.75 cc/min) and hydrogen (75 cc/min) to carry out a reaction. The maximum yield of benzene was 10. 9 % and the activity preservation ratio was 93 %.

Example 63

A reactor was charged with 0.3 g of the catalyst A, and was purged with helium and the temperature was increased to 200°C in a helium stream (10 cc/min) and held for 30 min. Subsequently, the gas was changed to a pre-contacting gas, which was a mixed gas of methane (6.75 cc/min), ethane (0.75 cc/min) and hydrogen (75 cc/min) and the temperature was increased to 650°C and held for 85 min. Then, only the hydrogen feeding was stopped and a reaction was initiated using methane and ethane as a reaction gas at the same flow rates at 650°C. The maximum yield of benzene was 8.7 % and the activity preservation ratio was 99 %.

Example 64

The procedure of Example 63 was repeated except that the holding temperature was 600°C and the holding time was 90 min in the pre-contacting step, and the reaction temperature was 600°C to carry out a reaction. The maximum yield of benzene was 7.9 % and the activity preservation ratio was 89 %.

INDUSTRIAL APPLICABILITY

According to the present invention, aromatic hydrocarbons can be produced industrially from lower hydrocarbons such as methane and the like in a high yield with a high activity preservation ratio.

**Claims**

1. A process for producing an aromatic hydrocarbon which process comprises:

   a pre-contacting step of allowing a molybdenum-containing solid catalyst to contact with a pre-contacting gas (G1) comprising at least one selected from a lower hydrocarbon and a hydrogen gas; and
   a reaction step of allowing the pre-contacted molybdenum-containing solid catalyst to contact with a raw material gas (G2) comprising a lower hydrocarbon essentially containing methane, to generate an aromatic hydrocarbon, wherein the starting temperature in the pre-contacting step is lower than the reaction temperature (Tr) in the reaction step, and the temperature during the pre-contacting step from the beginning to the end is not over the reaction temperature (Tr), and the molybdenum-containing solid catalyst comprises molybdenum and a zeolite having a ratio of silica to alumina of not more than 45.

2. The process for producing an aromatic hydrocarbon according to claim 1, wherein the starting temperature in the pre-contacting step is not higher than 350°C and the pre-contacting temperature is increased with passage of time.

3. The process for producing an aromatic hydrocarbon according to claim 1 or 2, wherein in the pre-contacting step, the contact of the molybdenum-containing solid catalyst and the pre-contacting gas (G1) is carried out with maintaining the temperature at a holding temperature (Th) which is not higher than the reaction temperature (Tr) for a certain period of time.

4. The process for producing an aromatic hydrocarbon according to claim 3, wherein in the pre-contacting step, the holding temperature (Th) is not lower than 400°C and not higher than the reaction temperature (Tr).

5. The process for producing an aromatic hydrocarbon according to claim 1 or 2, wherein when the temperature of the pre-contacting step reaches to the reaction temperature (Tr), the pre-contacting gas (G1) is changed to the raw material gas (G2) and the reaction step is initiated.

6. The process for producing an aromatic hydrocarbon according to any one of claims 1 to 5, wherein the pre-contacting gas (G1) is a mixed gas comprising hydrogen and a lower hydrocarbon essentially containing methane.

7. The process for producing an aromatic hydrocarbon according to claim 6, wherein the pre-contacting gas (G1) is a mixed gas comprising methane and hydrogen.

8. The process for producing an aromatic hydrocarbon according to claim 6 or 7, wherein:

   a) the mixed gas comprises 5 or more moles of hydrogen per 1 mole of methane; or
   b) the mixed gas comprises 10 or more moles of hydrogen per 1 mole of methane.

9. The process for producing an aromatic hydrocarbon according to any one of claims 1 to 8, wherein in the pre-contacting step, the flow rate of the pre-contacting gas (G1) satisfies at least one condition of a condition such that the flow rate of the pre-contacting gas (G1) is more than two times as high as the flow rate of the reaction gas (G2) in the reaction step and a condition such that the ratio (F/W) of the flow rate (F) of the pre-contacting gas (G1) to the catalyst weight (W) is not less than 50 cc/(g·min).

10. The process for producing an aromatic hydrocarbon according to any one of claims 1 to 9, wherein after the atmospheric gas of a catalyst layer is purged with an inert gas, the pre-contacting step is carried out.

11. The process for producing an aromatic hydrocarbon according to claim 10, wherein the temperature of the purging with an inert gas is not higher than 350ₒC.

12. The process for producing an aromatic hydrocarbon according to any one of claims 1 to 11, wherein the raw material gas (G2) does not contain hydrogen substantially.

13. The process for producing an aromatic hydrocarbon according to any one of claims 1 to 12, wherein the crystalline metallosilicate is an MFI type zeolite or an MWW type zeolite.

14. The process for producing an aromatic hydrocarbon according to claim 13, wherein the molybdenum is supported in an amount of 7 to 20 % by weight in the molybdenum-containing solid catalyst.

**Patentansprüche**

1. Verfahren zum Herstellen eines aromatischen Kohlenwasserstoffs, welches Verfahren umfasst:

     einen Vorkontaktierungsschritt des Erlaubens eines Molybdän enthaltenden Feststoffkatalysators mit einem Vorkontaktierungsgas (G1), umfassend mindestens eines, ausgewählt aus einem niederen Kohlenwasserstoff und einem Wasserstoffgas, zu kontaktieren; und
     einen Reaktionsschritt des Erlaubens des vorkontaktierten Molybdän enthaltenden Feststoffkatalysators mit einem Rohmaterialgas (G2), umfassend einen niederen Kohlenwasserstoff, der im Wesentlichen Methan enthält, zu kontaktieren, um einen aromatischen Kohlenwasserstoff zu erzeugen,
     wobei die Anfangstemperatur in dem Vorkontaktierungsschritt niedriger ist als die Reaktionstemperatur (Tr) in dem Reaktionsschritt, und die Temperatur während des Vorkontaktierungsschritts vom Anfang bis zum Ende nicht über der Reaktionstemperatur (Tr) liegt, und der Molybdän enthaltende Feststoffkatalysator Molybdän und einen Zeolith mit einem Verhältnis von Siliziumdioxid zu Aluminiumoxid von nicht mehr als 45 umfasst.

2. Verfahren zum Herstellen eines aromatischen Kohlenwasserstoffs nach Anspruch 1, wobei die Anfangstemperatur in dem Vorkontaktierungsschritt nicht höher ist als 350 °C und die Vorkontaktierungstemperatur im Verlauf der Zeit erhöht wird.

3. Verfahren zum Herstellen eines aromatischen Kohlenwasserstoffs nach Anspruch 1 oder 2, wobei in dem Vorkontaktierungsschritt der Kontakt des Molybdän enthaltenden Feststoffkatalysators und des Vorkontaktierungsgases (G1) unter Beibehalten der Temperatur bei einer Haltetemperatur (Th), die nicht höher ist als die Reaktionstemperatur (Tr), für einen bestimmten Zeitraum durchgeführt wird.

4. Verfahren zum Herstellen eines aromatischen Kohlenwasserstoffs nach Anspruch 3, wobei in dem Vorkontaktierungsschritt die Haltetemperatur (Th) nicht niedriger als 400 °C und nicht höher als die Reaktionstemperatur (Tr) ist.

5. Verfahren zum Herstellen eines aromatischen Kohlenwasserstoffs nach Anspruch 1 oder 2, wobei, wenn die Temperatur des Vorkontaktierungsschritts die Reaktionstemperatur (Tr) erreicht, das Vorkontaktierungsgas (G1) zu dem Rohmaterialgas (G2) gewechselt wird und der Reaktionsschritt initüert wird.

6. Verfahren zum Herstellen eines aromatischen Kohlenwasserstoffs nach einem beliebigen der Ansprüche 1 bis 5, wobei das Vorkontaktierungsgas (G1) ein gemischtes Gas ist, umfassend Wasserstoff und einen niederen Kohlenwasserstoff, der im Wesentlichen Methan enthält.

7. Verfahren zum Herstellen eines aromatischen Kohlenwasserstoffs nach Anspruch 6, wobei das Vorkontaktierungsgas (G1) ein gemischtes Gas ist, das Methan und Wasserstoff umfasst.

8. Verfahren zum Herstellen eines aromatischen Kohlenwasserstoffs nach Anspruch 6 oder 7, wobei:

a) das gemischte Gas 5 oder mehr Mol Wasserstoff pro 1 Mol Methan umfasst; oder
b) das gemischte Gas 10 oder mehr Mol Wasserstoff pro 1 Mol Methan umfasst.

9. Verfahren zum Herstellen eines aromatischen Kohlenwasserstoffs nach einem beliebigen der Ansprüche 1 bis 8, wobei in dem Vorkontaktierungsschritt die Flussrate des Vorkontaktierungsgases (G1) mindestens eine Bedingung einer Bedingung, sodass die Flussrate des Vorkontaktierungsgases (G1) mehr als zweimal so hoch ist wie die Flussrate des Reaktionsgases (G2) in dem Reaktionsschritt und einer Bedingung, sodass das Verhältnis (F/W) der Flussrate (F) des Vorkontaktierungsgases (G1) zu dem Katalysatorgewicht (W) nicht weniger als 50 cc/(g · min) beträgt, erfüllt.

10. Verfahren zum Herstellen eines aromatischen Kohlenwasserstoffs nach einem beliebigen der Ansprüche 1 bis 9, wobei nachdem das atmosphärische Gas einer Katalysatorschicht mit einem Inertgas ausgespült ist, der Vorkontaktierungsschritt durchgeführt wird.

11. Verfahren zum Herstellen eines aromatischen Kohlenwasserstoffs nach Anspruch 10, wobei die Temperatur des Spülens mit einem Inertgas nicht höher als 350 °C ist.

12. Verfahren zum Herstellen eines aromatischen Kohlenwasserstoffs nach einem beliebigen der Ansprüche 1 bis 11, wobei das Rohmaterialgas (G2) im Wesentlichen keinen Wasserstoff enthält.

13. Verfahren zum Herstellen eines aromatischen Kohlenwasserstoffs nach einem beliebigen der Ansprüche 1 bis 12, wobei das kristalline Metallosilikat ein MFI-Typ Zeolith oder ein MWW-Typ Zeolith ist.

14. Verfahren zum Herstellen eines aromatischen Kohlenwasserstoffs nach Anspruch 13, wobei das Molybdän in einer Menge von 7 bis 20 Gewichtsprozent in dem Molybdän enthaltenden Feststoffkatalysator geträgert ist.

**Revendications**

1. Procédé pour produire un hydrocarbure aromatique, lequel procédé comporte :

une étape de mise en précontact, consistant à permettre à un catalyseur solide contenant du molybdène de venir au contact d'un gaz de mise en pré-contact (G1) comprenant au moins un gaz choisi entre un hydrocarbure inférieur et de l'hydrogène gazeux ; et
une étape de réaction, consistant à permettre au catalyseur solide mis en précontact contenant du molybdène de venir au contact d'une matière gazeuse brute (G2) comprenant un hydrocarbure inférieur contenant essentiellement du méthane, afin de générer un hydrocarbure aromatique,
la température initiale lors de l'étape de mise en précontact étant inférieure à la température de réaction (Tr) lors de l'étape de réaction, et la température pendant l'étape de mise en précontact, du début à la fin, n'étant pas supérieure à la température de réaction (Tr), et le catalyseur solide contenant du molybdène comprend du moblybdène et une zéolite à proportion de silice par rapport à l'alumine non supérieure à 45.

2. Procédé pour produire un hydrocarbure aromatique selon la revendication 1, dans lequel la température initiale lors de l'étape de mise en précontact n'est pas supérieure à 350°C et la température de mise en précontact est amenée à augmenter au fil du temps.

3. Procédé pour produire un hydrocarbure aromatique selon la revendication 1 ou 2, dans lequel, lors de l'étape de mise en précontact, le contact entre le catalyseur solide contenant du molybdène et de gaz de mise en précontact (G1) est réalisé en conservant la température pendant un certain laps de temps à une température de maintien (Th) non supérieure à la température de réaction (Tr).

4. Procédé pour produire un hydrocarbure aromatique selon la revendication 3, dans lequel, lors de l'étape de mise en précontact, la température de maintien (Th) n'est pas inférieure à 400°C et n'est pas supérieure à la température de réaction (Tr).

5. Procédé pour produire un hydrocarbure aromatique selon la revendication 1 ou 2, dans lequel, quand la température de l'étape de mise en précontact atteint la température de réaction (Tr), la matière gazeuse brute (G2) est substituée au gaz de mise en précontact (G1) et l'étape de réaction est lancée.

6. Procédé pour produire un hydrocarbure aromatique selon l'une quelconque des revendications 1 à 5, dans lequel le gaz de mise en précontact (G1) est un mélange gazeux comprenant de l'hydrogène et un hydrocarbure inférieur contenant essentiellement du méthane.

7. Procédé pour produire un hydrocarbure aromatique selon la revendication 6, dans lequel le gaz de mise en précontact (G1) est un mélange gazeux comprenant du méthane et de l'hydrogène.

8. Procédé pour produire un hydrocarbure aromatique selon la revendication 6 ou 7, dans lequel, dans lequel :

   a) le mélange gazeux comprend 5 moles ou plus d'hydrogène pour 1 mole de l'éthane ; ou
   b) le mélange gazeux comprend 10 moles ou plus d'hydrogène pour 1 mole de méthane.

9. Procédé pour produire un hydrocarbure aromatique selon l'une quelconque des revendications 1 à 8, dans lequel, lors de l'étape de mise en précontact, le débit du gaz de mise en précontact (G1) respecte au moins une condition parmi une condition telle que le débit du gaz de mise en précontact (G1) soit supérieur au double du débit du gaz de réaction (G2) lors de l'étape de réaction une condition telle que le rapport (F/W) du débit (F) du gaz de mise en précontact (G1) au poids du catalyseur ne soit pas inférieur à 50 $cm^3$/(g·min).

10. Procédé pour produire un hydrocarbure aromatique selon l'une quelconque des revendications 1 à 9, dans lequel, après que le gaz atmosphérique d'une couche de catalyseur a été purgé à l'aide d'un gaz inerte, l'étape de mise en précontact est réalisée.

11. Procédé pour produire un hydrocarbure aromatique selon la revendication 10, dans lequel la température pour l'opération de purge à l'aide d'un gaz inerte n'est pas supérieure à 350°C.

12. Procédé pour produire un hydrocarbure aromatique selon l'une quelconque des revendications 1 à 11, dans lequel la matière gazeuse brute (G2) ne contient sensiblement pas d'hydrogène.

13. Procédé pour produire un hydrocarbure aromatique selon l'une quelconque des revendications 1 à 12, dans lequel le métallosilicate cristallin est une zéolite de type MFI ou une zéolite de type MWW.

14. Procédé pour produire un hydrocarbure aromatique selon la revendication 13, dans lequel le molybdène est supporté à raison de 7 à 20 % en poids dans le catalyseur solide contenant du molybdène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US P4822944 A
- US P5336825 A
- US P4239658 A
- US P4507517 A
- US P4567311 A
- US P4734537 A
- WO 9851409 A
- EP 216491 B
- US P5557022 A
- EP 228267 B
- JP H101998272366 A
- US P5026937 A
- US P5936135 A
- US P6239057 B
- JP 2001334151 A
- JP 2002336704 A
- JP 2004097891 A
- JP 2005144360 A
- JP H11199947606 A
- WO 0210099 A
- WO 200528105 A
- JP 2005254120 A
- JP 2005254121 A
- JP H11199960514 A
- JP 2004269398 A
- JP 2005255605 A
- JP 2005343879 A
- WO 200611568 A
- JP 2003026613 A
- WO 200683409 A
- WO 200687971 A

### Non-patent literature cited in the description

- *Science,* 1966, vol. 153, 1393
- *Russian Chemical Bulletin,* 1982, vol. 31 (4), 847
- *Russian Chemical Bulletin,* 1989, vol. 38 (3), 680
- *Catalysis Letters,* 1993, vol. 21, 35
- *Catalysis Letters,* 1996, vol. 39, 169
- *Applied Catalysis A,* 1997, vol. 152, 173
- *Reaction Kinetics and Catalysis Letters,* 1997, vol. 61, 391
- *Journal of Catalysis,* 1997, vol. 170, 11
- *Journal of Natural Gas Chemistry,* 2004, vol. 13, 36
- **K. OKUMURA et al.** *Journal of Catalysis,* 2002, vol. 206, 23